# EUROPÄISCHE PATENTSCHRIFT

(11) **EP 2 269 058 B1**
(45) Veröffentlichungstag und Bekanntmachung des Hinweises auf die Patenterteilung: **13.02.2013**
(21) Anmeldenummer: 09734577.1
(22) Anmeldetag: 22.04.2009
(51) Int. Cl.: G01N 33/28

(54) **VERFAHREN ZUR CHARAKTERISIERUNG DER KLOPFFESTIGKEIT VON KRAFTSTOFFEN**
METHOD FOR CHARACTERISING THE KNOCK-RESISTANCE OF FUELS
PROCÉDÉ POUR CARACTÉRISER LE POUVOIR ANTIDÉTONANT DE CARBURANTS

(30) Priorität: 22.04.2008 DE 102008001306
(43) Veröffentlichungstag der Anmeldung: 05.01.2011
(73) Patentinhaber: Rofa Laboratory & Process Analyzers, 3420 Kritzendorf (AT)
(72) Erfinder: HUBER, Karl, 85072 Eichstätt (DE); HAUBER, Johann, 86633 Neuburg (DE)
(74) Vertreter: Bergmeier, Werner
(86) Internationale Anmeldenummer: PCT/EP2009/054844
(87) Internationale Veröffentlichungsnummer: WO 2009/130254

(56) Entgegenhaltungen:
- EP-A- 0 039 881
- WO-A2-2008/024663
- US-A- 5 386 722
- RAHMOUNI C ET AL: "Knock rating of gaseous fuels in a single cylinder spark ignition engine" FUEL, IPC SCIENCE AND TECHNOLOGY PRESS, GUILDFORD, GB, Bd. 83, Nr. 3, 1. Februar 2004 (2004-02-01), Seiten 327-336, XP004467282 ISSN: 0016-2361
- MEROLA ET AL: "Knock investigation by flame and radical species detection in spark ignition engine for different fuels" ENERGY CONVERSION AND MANAGEMENT, ELSEVIER SCIENCE PUBLISHERS, OXFORD, GB, Bd. 48, Nr. 11, 3. Oktober 2007 (2007-10-03), Seiten 2897-2910, XP022283941 ISSN: 0196-8904
- KLAAS BURGDORF ET AL: "Comparison of Cylinder Pressure Based Knock Detection Methods", SAE TECHNICAL PAPER SERIES, SOCIETY OF AUTOMOTIVE ENGINEERS, WARRENDALE, PA, US, 1 October 1997 (1997-10-01), pages 133-150, XP008133820, ISSN: 0148-7191, DOI: DOI:10.4271/972932
- HUDSON C ET AL: "Knock measurement for fuel evaluation in spark ignition engines", FUEL, IPC SCIENCE AND TECHNOLOGY PRESS, GUILDFORD, GB, vol. 80, no. 3, 1 February 2001 (2001-02-01), pages 395-407, XP004286395, ISSN: 0016-2361, DOI: DOI:10.1016/S0016-2361(00)00080-6
- D. DIMITROV; F. CHMELA; A. WIMMER: 'Eine Methode zur Vorausberechnung des Klopfverhaltens von Gasmotoren', [Online] 03 Juni 2005, 4. Dessauer Gasmotoren-Konferenz Gefunden im Internet: <URL:http://www.wtz.de/sites/05_gmk/GMK_050 4-0109.html> [gefunden am 2011-08-08]

## Beschreibung

Die Erfindung betrifft ein Verfahren zur Charakterisierung der Klopffestigkeit von Kraftstoffen mit Hilfe eines Prüfmotors.

In der Norm DIN EN 228 sind die Kennwerte und Eigenschaften als Mindestanforderungen für bleifreie Ottokraftstoffe festgelegt. Tabelle 1 zeigt einen Auszug wesentlicher Kraftstoffkennwerte.

| | | **Anforderungen nach DIN EN 228** | | |
|---|---|---|---|---|
| **Kennwert** | **Einheit** | **SuperPlus** | **Super** | **Normal** |
| Dichte bei 15 °C | Kg/m³ | 720-775 | | |
| Klopffestigkeit | | | | |
| ROZ | | min 98 | min 95 | min 91 |
| MOZ | | min 88 | min 85 | min 82.5 |
| Bleigehalt | mg/l | max 5 | | |
| Siedeverlauf * | %(V/V) | | | |
| Venlampfte Menge (Klasse A) | | | | |
| bei 70 °C, E70 | | 20-48 | | |
| bei 100 °C, E100 | | 46-71 | | |
| bei 150 °C, E150 | | min 75 | | |
| Verdamptte Menge (Klasse D/D1) | | | | |
| bei 70 °C, E70 | | 22-50 | | |
| bei 100 °C E100 | | 46-71 | | |
| bei 150 °C, E150 | | min 75 | | |
| Siedeendpunk1 PBP (Klasse A/D/D1) | °C | max 210 | | |
| Flüchtigkeitskennziffer VI.1** | | | | |
| (VI.1 10 × VP + 7 × E70) | | | | |
| Klasse D1 | Index | max 1150 | | |
| Destillationsrückstand | %(V/V) | max 2 | | |
| Dampfdruck (DVPE) | kPa | | | |
| Klasse A | | 45.0-60.0 | | |
| Klasse D/D1 | | 60.0-90.0 | | |
| Abdampfrückstand | mg/100 ml | max 5 | | |
| genzolgehält | % (V/V) | max 1 | | |
| Schwefelgehalt | mg/kg | max 150 | | |
| Oxidationsstabilität | min | min 360 | | |
| Kapferkorroion | Korrosionsgrad | max 1 | | |

| | | | | |
|---|---|---|---|---|
| * Klasse A:01.05 - 30.09 (Sommer) Klasse D: 16.11 - 15.03 (Winter) Klasse D1: 16.03 - 30.04./01. 10. - 15. 11 (Übergang) ** Vapor-Lock-Index | | | | |

Von besonderer Bedeutung ist dabei die Klopffestigkeit, die mit zwei Kennzahlen, der Motor-Oktanzahl (MOZ) und der Research-Oktanzahl (ROZ) beschrieben wird. Zur Erläuterung ist zu sagen, dass klopfende Verbrennungen in jedem Ottomotor auftreten können und bei entsprechender Intensität kapitale Motorschäden verursachen. Die Motorentwickler sind deshalb angehalten, die unregelmäßige Verbrennungserscheinung des Klopfens zu verhindern, d.h. Klopfregelsysteme in die Motorsteuerung zu integrieren und den Motor konstruktiv auf die Klopffestigkeit des Kraftstoffs vorzubereiten. Hohe Oktanzahlen erlauben eine höhere Leistung bei gleichzeitig höherem Motorenwirkungsgrad und damit einem niedrigeren Verbrauch. Aus diesen Gründen können mit hochoktanigen Kraftstoffen auch höherer Preise erzielt werden, obwohl die Kraftstoffe annähernd die gleichen Energieinhalte (Heizwerte) aufweisen.

Die Ermittlung der Oktanzahlen wird heute weltweit empirisch und nach genormten Verfahren in den Laboren der Kraftstoffproduzenten durchgeführt. Zum Einsatz kommen dabei spezielle Einzylinder-Prüfmotoren, deren Verdichtungsverhältnis variable ist und auf die jeweilige Kraftstoffqualität eingestellt werden kann. Ziel ist es, den zu prüfenden Kraftstoff hinsichtlich seiner Klopfintensität mit Kraftstoffen bekannter Oktanzahlen zu vergleichen und ggf. durch Interpolation die Oktanzahlen zu ermitteln. Willkürlich wurde in der Norm Iso-Oktan die Oktanzahl 100 und n-Heptan die Oktanzahl 0 zugeordnet. Durch Mischen dieser Komponenten kann jeweils ein Kraftstoff hergestellt werden, der die gleiche Klopfintensität wie der zu prüfende Kraftstoff aufweist. Die gesuchte Oktanzahl entspricht dann dem volumetrischen Anteil an Iso-Oktan der Kraftstoffmischung. Hinsichtlich der Prüfbedingungen wird zwischen MOZ und ROZ unterschieden, wobei alle anderen Verfahrensschritte übereinstimmen und auch dieselbe Messtechnik und derselbe Prüfmotor verwendet werden.

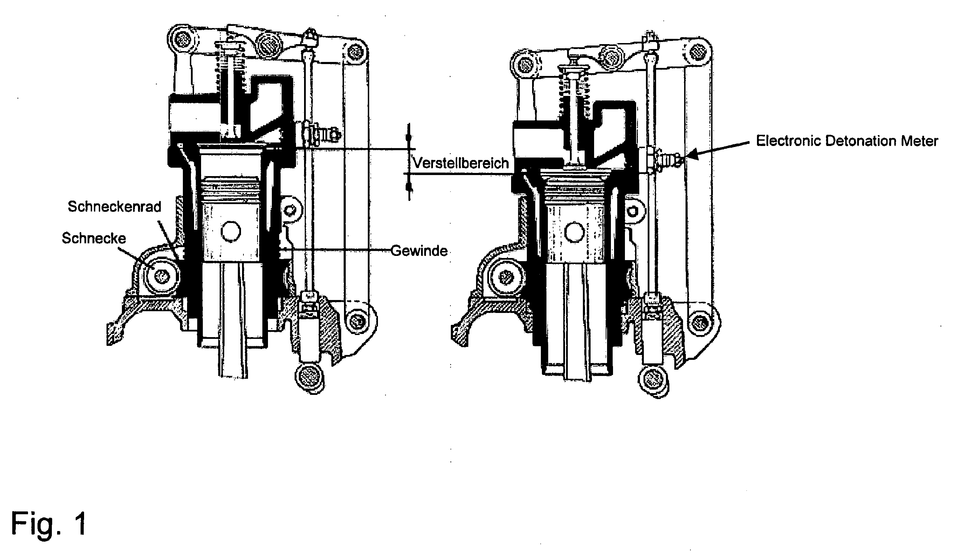

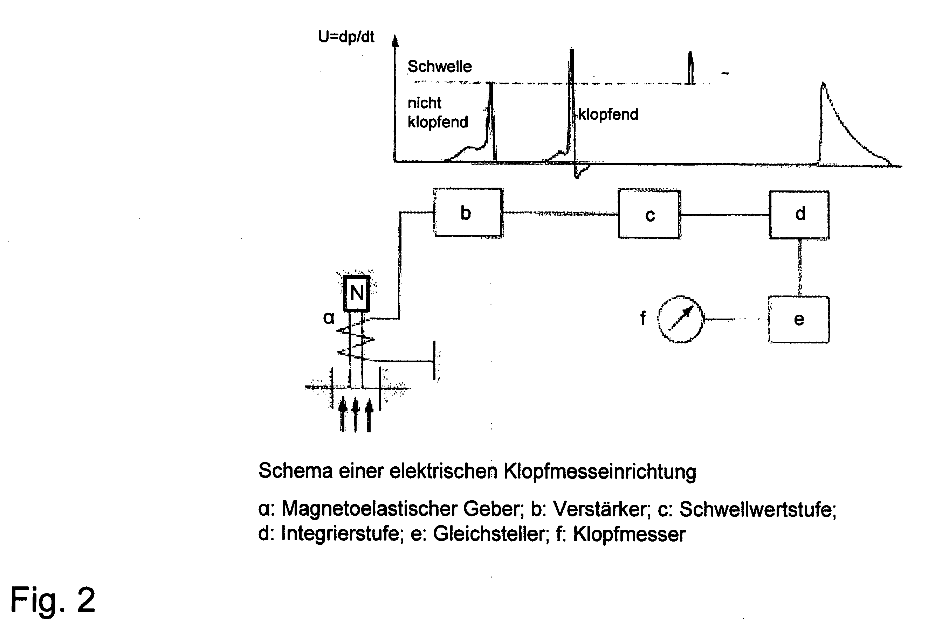

Das Maß der Klopfintensität wird jeweils über einen elektrischen Sensor (Electronic-Detonation-Meter), der in den Brennraum des Motors eingeschraubt ist (Fig. 1), erzeugt und über eine analoge Schaltung (Fig. 2) auf ein Zeigerinstrument (Knock-Meter) zur Anzeige gebracht. Berechnungen hinsichtlich Klopfintensität und Häufigkeit werden mit den Messdaten nicht durchgeführt. Aus eigenen Untersuchungen ist bekannt, dass Kraftstoffe gleicher Oktanzahlen durchaus unterschiedliches Klopfverhalten hinsichtlich Intensität und Häufigkeit aufweisen können. Bei professioneller Anwendung und entsprechender Erfahrung kann mit diesem Verfahren eine Genauigkeit von höchstens + 0,2 Oktanzahlen erreicht werden. Die Bedienung erfolgt manuell und dauert 20 - 30 min. pro Oktanzahl.

Hudson et al. Fuel 80 (2001) 395 beschreiben einen Ansatz zur Charakterisierung der Klopffestigkeit mittels statistischer Analyse von Klopf-Ereignissen.

Es gab und gibt immer wieder Versuche, die Oktanzahlbestimmung zu berechnen oder mit einem anderen Gerät, d.h. außermotorisch, durchzuführen. Leider ist dies bis heute nicht mit der gewünschten Genauigkeit gelungen. Bei bekannter Zusammensetzung des Treibstoffes kann mittels gaschromatographischer Analyse oder Infrarot-Spektroskopie eine annähernd exakte Bestimmung erreicht werden. Diese Methoden kommen aber nur in Raffinerien zur Anwendung, weil diese die Zusammensetzung des Treibstoffes genau kennen. Eine grundlegende Verbesserung der Vorrichtung und des Verfahrens wurde nicht gefunden.

In der Beurteilung der gültigen Verfahren zur Oktanzahlbestimmung sind folgende Probleme festzustellen:
- Das Verfahren ist hinsichtlich seiner Genauigkeit verbesserungsbedürftig.
- Das Verfahren ist zeitintensiv, nicht automatisierbar und erlaubt keine Online-Anzeige.
- Bei dem Verfahren wird die Klopfintensität nach analoger Bearbeitung des Messsignals direkt angezeigt. Eine genaue Berechnung hinsichtlich Klopfintensität und Häufigkeit findet nicht statt.
- Ob die ermittelten Oktanzahlen tatsächlich die Klopffestigkeit von Kraftstoffen wiedergeben, die von aktuellen Verbrennungsmotoren gefordert werden, ist in Frage zu stellen.

Es ist nun Aufgabe der Erfindung, ein Verfahren vorzuschlagen, mit dessen Hilfe eine schnelle und zuverlässige Charakterisierung der Klopffestigkeit von Kraftstoffen möglich ist.

Diese Aufgabe darch das Verfahren nach Anspruch 1 gelöst

Durch den Einsatz moderner Mess- und Analysetechnik ist es möglich, die im Prüfmotor ablaufenden klopfenden Verbrennungen hinsichtlich verschiedener Kenngrößen, wie beispielsweise der Klopfintensität, der Klopfdruckamplitude, des Spitzendrucks, der Druckanstiegsgeschwindigkeit, der Frequenz sowie der Häufigkeitsverteilungen dieser Kenngrößen exakt zu charakterisieren. Die Vorgehensweise und die notwendig Messtechnik sind nachfolgend beschrieben.

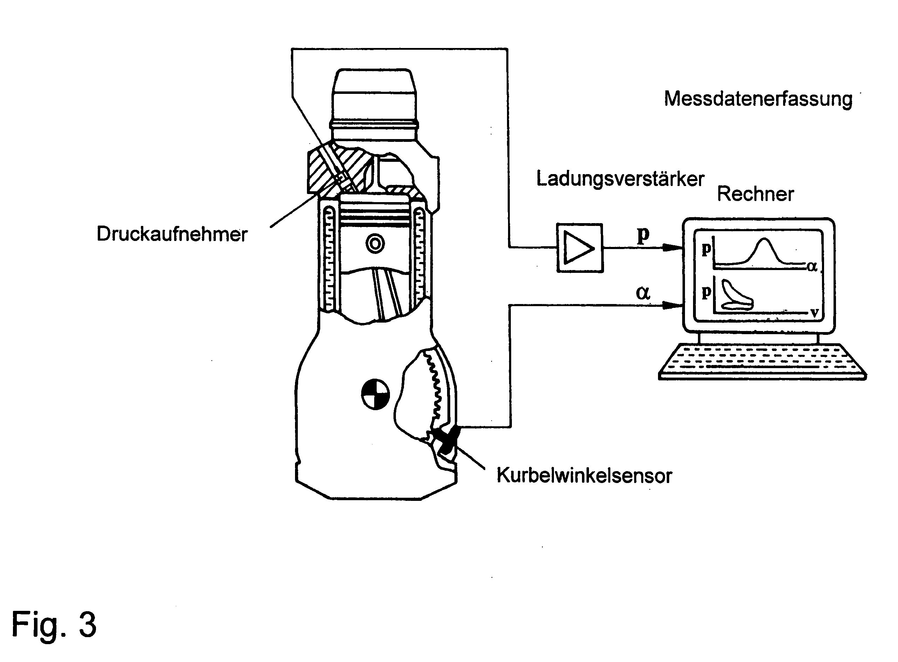

Anstelle des Electronic-Detonation-Meter wird hierbei ein piezoelektrischer Drucksensor eingebaut, wie er üblicherweise bei Forschungs- und Entwicklungsaufgaben der Motorenentwickler zum Einsatz kommt. Das Signal wird verstärkt und in ein dem Zylinderdruck (p) proportionales Spannungssignal umgewandelt (Fig. 3). Das Spannungssignal wird einer schnellen Messdatenerfassung zugeführt, digitalisiert, weiterverarbeitet und abgespeichert.

In diesem Zusammenhang kann es von Vorteil sein, wenn die Drucksignale vor dem Vergleich mit den entsprechenden Signalen eines oder mehrerer Normkraftstoffe mit Hilfe einer Signalfilterung mit Bandpass, insbesondere im Bereich von 3 bis 15 kHz und/oder mit Hochpass ab 3 kHz, gefiltert werden.

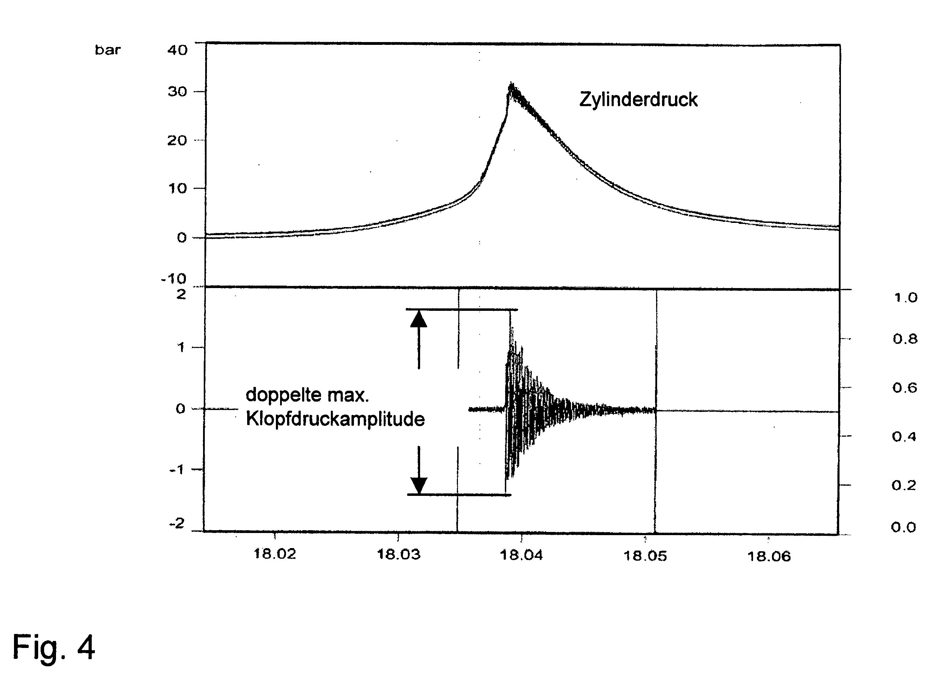

Gleichzeitig ist es von Vorteil, wenn der zeitliche Verlauf des Kurbelwinkels (α; s. Fig. 3) des Prüfmotors während der Verbrennung mit Hilfe eines Kurbelwinkelsensors ermittelt wird. Hierdurch lassen sich die ermittelten Drucksignale entweder als Funktion der Zeit oder aber auch des Kurbelwinkels und damit z.B. in Abhängigkeit des Zündzeitpunkts analysieren. Eine entsprechende Darstellung des Zylinderdrucks bzw. der entsprechenden Klopfdruckamplituden in Abhängigkeit der Zeit und damit auch des Kurbelwinkels zeigt Fig. 4

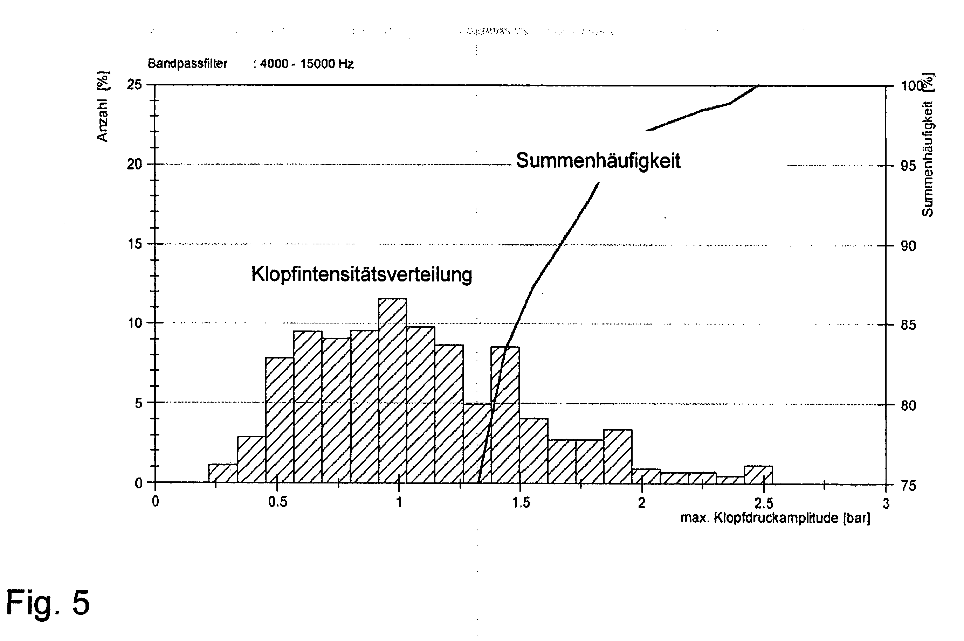

Als Ergebnis können die Ktopfintensität oder die sonstigen oben genannten aus dem Verlauf des Zylinderdrucks ableitbaren Kenngrößen und deren Verteilung als Histogramm, als Summenhäufigkeit oder als einzelne Kennzahl zur Beschreibung des Klopfens ausgegeben werden (Fig. 5). Auch ist die Gegenüberstellung der einzelnen Kenngrößen möglich, da auch hieraus wichtige Erkenntnisse über das Klopfverhalten des untersuchten Kraftstoffs gewonnen werden können. Hierbei sei darauf hingewiesen, dass bei der Interpretation der ermittelten Daten auch die jeweiligen Betriebsbedingungen des Prüfmotors berücksichtigt werden müssen, um eine Verfälschung der resultierenden Kenngrößen zu vermeiden.

Ein Vergleich der entsprechenden Ergebnisse mit den analog erhaltenen Ergebnissen üblicher Normkraftstoffe wie Iso-Oktan und/oder n-Heptan führt dann zu einer oder mehreren Kennzahlen, welche die Klopffestigkeit des zu prüfenden Kraftstoffs exakt beschreiben.

Die jeweiligen Kennzahlen, die auf Basis des zeitlichen Verlaufs des Zylinderdrucksignals des Prüfmotors erhalten werden, können dabei entweder im Verlauf eines einzelnen Arbeitsspiels des Prüfmotors oder auch aus der statistischen Auswertung der Drucksignale mehrerer Arbeitsspiele erhalten werden.

Zudem ist es auch von Vorteil, wenn das Verdichtungsverhältnis des Prüfmotors auf Basis des Zylinderdrucks bei einem definierten Kurbelwinkel berechnet wird, wobei der Kurbelwinkel als Maß des Zündzeitpunkts dient. Da das Klopfverhalten eines Kraftstoffs unter anderem vom Verdichtungsverhältnis des Prüfmotors abhängt, ist es von Vorteil, diese Kenngröße beim Vergleich der Drucksignale des untersuchten Kraftstoffs mit denen des Norm-Kraftstoffs zu berücksichtigen.

Des weiteren ist es von erheblichem Vorteil, wenn die gemessenen Zylinderdrucksignale sowie entsprechend zusätzlich bekannte Daten des untersuchten Kraftstoffes, wie beispielsweise dessen genaue chemische Zusammensetzung, in einer Datenbank gespeichert werden. Diese Messwerte liefern schließlich die Basis für zukünftige Bewertungen von neuen oder bisher nicht untersuchten Kraftstoffen, wobei insbesondere unter Einhaltung genormter Verfahrensschritte schnelle und zuverlässige Rückschlüsse auf das Klopfverhalten eines zu untersuchenden Kraftstoffs erhalten werden können. Eine Analyse von Norm-Kraftstoffen ist somit bei einem entsprechenden Datenbestand nicht mehr in jedem Fall notwendig.

Die Verfahrensschritte sind beispielsweise wie folgt:
- Zeitbasierte Messung des Zylinderdrucksignals (Abtastfrequenz 100 kHz).
- Bandpassfilterung des gemessenen Drucksignals (3 - 15 kHz).
- Ermittlung der maximalen Amplitudenhöhe des gefilterten Drucksignals je Arbeitsspiel (vorteilhaft mit einem Signalprozessor zu realisieren).
- Auswerten der Amplitudenhöhe und deren Häufigkeit in einem einstellbaren Zeitfenster (300 Arbeitsspiele, Intensitätsverteilung oder Summenhäufigkeit).
- Korrelation der Ergebnisse mit den bekannten Normkraftstoffen bzw. deren Mischungen.
- Anzeige und Abspeichern der Korrelationsindizes als Maß für die Klopffestigkeit.

Als wesentliche Vorteile der Vorrichtung und des Verfahrens sind zu nennen:
- höhere Genauigkeit, insbesondere durch Einsatz von Präzisionsmesstechnik und statistischer Analyse.
- genauere Bewertung der Klopfvorgänge durch Analyse von Klopfintensität und Häufigkeit.
- Möglichkeit zur Automatisierung.
- Online-Anzeige der Klopffestigkeitskennzahl.
- Automatischer oder halbautomatische Ablauf.
- Entfall der Messung mit Normkraftstoffen, sobald die Ergebnisse in einer Datenbank zur Verfügung gestellt worden sind.
- Das Verdichtungsverhältnis kann exakt aus dem Druckverlauf vor Zündzeitpunkt berechnet werden.

So kann es durchaus von Vorteil sein, wenn der Druck und/oder die Temperatur des Verbrennungsgemisches aus Kraftstoff und Verbrennungsgas, insbesondere Verbrennungsluft, sowie die Verweildauer des Verbrennungsgemisches, insbesondere in Form einer hieraus berechneten Kennziffer, berücksichtigt werden. Auch kann es vorteilhaft sein, wenn der Zündzeitpunkt sowie der Klopfbeginn des Verbrennungsgemisches, insbesondere mit Hilfe eines Sensors, erfasst werden und die dazwischenliegende Zeitdifferenz und/oder die Differenz der jeweiligen Kurbelwinkel des Prüfmotors berücksichtigt werden. Auf diese Weise lässt sich der zu analysierende Zeitraum besonders genau festlegen, resultierend in einer besonders zuverlässigen Versuchsdurchführung. Des Weiteren kann auch der Brennverlauf (Wärmeeintrag durch Verbrennung) eines einzelnen oder mehrerer Arbeitsspiele des Prüfmotors und hieraus der Zündverzug, die Vorentflammung, die maximale Brenngeschwindigkeit und/oder die Restmenge des Verbrennungsgemisches aus Kraftstoff und Verbrennungsgas, insbesondere Verbrennungsluft, im Zeitpunkt des Klopfbeginns ermittelt werden. Auch kann der Brennverlauf eines einzelnen oder mehrerer Arbeitsspiele des Prüfmotors ermittelt und wenigstens ein vorher definierter Wert des Brennverlaufs, wie beispielsweise der Brennbeginn, mit Hilfe wenigstens eines Sensors, insbesondere eines Sensors zur Messung des lonenstroms innerhalb des Prüfmotors, eines Sensors zur Messung des Körperschalls des Prüfmotors und/oder eines optischen Sensors, ermittelt werden. Auf diese Weise lässt sich der Brennbeginn besonders genau feststellen, so dass der Zeitraum für die entsprechende Zylinderdruckmessung möglichst zuverlässig bestimmt werden kann. Ebenso ist es von enormen Vorteil, wenn die Charakterisierung der Klopffestigkeit eine statistische Auswertung von Messwerten, insbesondere der Drucksignale, enthält, wobei die statistische Auswertung die Aufzeichnung von Messwerten, insbesondere der Drucksignale, eines oder mehrerer Arbeitsspiele, beispielsweise zwischen 200 und 500 Arbeitsspiele, des Prüfmotors, insbesondere in einem definierten Betriebspunkt des Prüfmotors, umfasst. Auch ist es von Vorteil, wenn die statistische Auswertung die Berechung von Mittelwerten der Messwerte umfasst, so dass sich etwaige Messschwankungen nur minimal auf die Charakterisierung der Klopffestigkeit auswirken.

## Patentansprüche

1. Verfahren zur Charakterisierung der Klopffestigkeit eines Kraftstoffs mit Hilfe eines Prüfmotors, wobei
- der zeitliche Verlauf des Zylinderdrucks des Prüfmotors während der Verbrennung des Kraftstoffs im Prüfmotor jeweils separat für mehrere Arbeitsspiele des Prüfmotors, insbesondere mit Hilfe eines piezoelektrischen Drucksensors, detektiert wird,
- dass die ermittelten Druckverläufe separat bezüglich einer definierten Kenngröße, beispielsweise der maximalen Druckamplitude, ausgewertet werden, **dadurch gekennzeichnet, dass**
- dass die jeweiligen Kenngrößen der einzelnen Arbeitsspiele bezüglich ihrer Häufigkeitsverteilung ausgewertet werden,
- dass die detektierten Drucksignale auf Basis der ermittelten Häufigkeitsverteilung mit den entsprechenden Drucksignalen wenigstens eines Norm-Kraftstoffs bekannter Klopffestigkeit verglichen werden, und
- dass aus dem Vergleich der Drucksignale wenigstens eine Kennzahl ermittelt wird, welche als Maß für die Klopffestigkeit des Kraftstoffs dient.

2. Verfahren nach Anspruch 1, **dadurch gekennzeichnet, dass** als Norm-Kraftstoff iso-Oktan und/oder n-Heptan verwendet werden.

3. Verfahren nach einem oder mehreren der vorangegangenen Ansprüche, **dadurch gekennzeichnet, dass** der Druck und/oder die Temperatur des Verbrennungsgemisches aus Kraftstoff und Verbrennungsgas, insbesondere Verbrennungsluft, des Prüfmotors sowie die Verweildauer des Verbrennungsgemisches, insbesondere in Form einer hieraus berechneten Kennziffer, berücksichtigt werden.

4. Verfahren nach einem oder mehreren der vorangegangenen Ansprüche, **dadurch gekennzeichnet, dass** die Drucksignale vor dem Vergleich mit Hilfe einer Signalfilterung mit Bandpass, insbesondere im Bereich von 3 bis 15 kHz und/oder mit Hochpass ab 3 kHz, gefiltert werden.

5. Verfahren nach einem oder mehreren der vorangegangenen Ansprüche, **dadurch gekennzeichnet, dass** der Zündzeitpunkt sowie der Klopfbeginn des Verbrennungsgemisches aus Kraftstoff und Verbrennungsgas, insbesondere Verbrennungsluft, insbesondere mit Hilfe eines Sensors, erfasst werden und die dazwischenliegende Zeitdifferenz und/oder die Differenz der jeweiligen Kurbelwinkel des Prüfmotors berücksichtigt werden.

6. Verfahren nach einem oder mehreren der vorangegangenen Ansprüche, **dadurch gekennzeichnet, dass** die Drucksignale auf Basis ihrer Klopfdruckamplitude, ihres Spitzendrucks, ihrer Druckanstiegsgeschwindigkeit, ihrer Frequenz und/oder ihrer Klopfintensität miteinander verglichen werden.

7. Verfahren nach einem oder mehreren der vorangegangenen Ansprüche, **dadurch gekennzeichnet, dass** der Brennverlauf eines einzelnen oder mehrerer, insbesondere gemittelter, Arbeitsspiele des Prüfmotors ermittelt und hieraus der Zündverzug, die Vorentflammung, die maximale Brenngeschwindigkeit und/oder die Restmenge des Verbrennungsgemisches aus Kraftstoff und Verbrennungsgas, insbesondere Verbrennungsluft, im Zeitpunkt des Klopfbeginns bestimmt und bei der Charakterisierung der Klopffestigkeit berücksichtigt wird.

8. Verfahren nach einem oder mehreren der vorangegangenen Ansprüche, **dadurch gekennzeichnet, dass** der Brennverlauf eines einzelnen oder mehrerer Arbeitsspiele des Prüfmotors ermittelt wird und wenigstens ein vorher definierter Wert des Brennverlaufs, wie beispielsweise der Brennbeginn, mit Hilfe wenigstens eines Sensors, insbesondere eines Sensors zur Messung des Ionenstroms innerhalb des Prüfmotors, eines Sensors zur Messung des Körperschalls des Prüfmotors und/oder eines optischen Sensors, ermittelt und bei der Charakterisierung der Klopffestigkeit berücksichtigt wird.

9. Verfahren nach einem oder mehreren der vorangegangenen Ansprüche, **dadurch gekennzeichnet, dass** die Charakterisierung der Klopffestigkeit eine statistische Auswertung der Drucksignale enthält, wobei die statistische Auswertung die Ermittlung der Häufigkeitsverteilung der Drucksignale, Klopfdruckamplituden, Spitzendrücke, Druckanstiegsgeschwindigkeiten, Frequenz und/oder Klopfintensität umfasst.

10. Verfahren nach einem oder mehreren der vorangegangenen Ansprüche, **dadurch gekennzeichnet, dass** der zeitliche Verlauf des Kurbelwinkels des Prüfmotors während der Verbrennung mit Hilfe eines Kurbelwinkelsensors ermittelt wird.

11. Verfahren nach einem oder mehreren der vorangegangenen Ansprüche, **dadurch gekennzeichnet, dass** das Verdichtungsverhältnis des Prüfmotors auf Basis des Zylinderdrucks bei einem definierten Kurbelwinkel berechnet wird.

12. Verfahren nach einem oder mehreren der vorangegangenen Ansprüche, **dadurch gekennzeichnet, dass** die Drucksignale unter Berücksichtigung der Masse des eingespritzten Kraftstoffs und/oder des Verbrennungsgases, insbesondere der Verbrennungsluft, im Zylinder, unter Berücksichtigung der Ansaugtemperatur des Verbrennungsgases und/oder des Gemisches aus Verbrennungsgas und Kraftstoff, unter Berücksichtigung des Ansaugdrucks des Verbrennungsgases und/oder des Gemisches aus Verbrennungsgas und Kraftstoff und/oder unter Berücksichtigung der Drehzahl des Prüfmotors miteinander verglichen werden.

13. Verfahren nach einem oder mehreren der vorangegangenen Ansprüche, **dadurch gekennzeichnet, dass** die ermittelten Drucksignale, insbesondere in Abhängigkeit des Kurbelwinkels und/oder des Verdichtungsverhältnisses, in eine Datenbank aufgenommen werden.

## Claims

1. A process for characterizing the knock resistance of a fuel using a test engine,
- the time curve of the cylinder pressure of the test engine being detected separately for a plurality of working cycles of the test engine during the combustion of the fuel in the test engine, particularly by means of a piezoelectric pressure sensor,
- the derived pressure curves being analyzed separately with respect to a defined characteristic parameter, such as the maximum pressure amplitude, **characterized in that**
- each of the characteristic parameters of the individual working cycles is analyzed with respect to the frequency distribution thereof,
- the detected pressure signals are compared with the corresponding pressure signals of at least one standardized fuel of known knock resistance on the basis of the derived frequency distribution , and
- at least one characteristic value serving as a measure of the knock resistance of the fuel is derived from the comparison of the pressure signals.

2. The method according to claim 1, **characterized in that** isooctane and/or n-heptane is used as the standardized fuel.

3. The method according to one or more of the preceding claims, **characterized in that** the pressure and/or the temperature of the combustion mixture of fuel and combustion gas, particularly combustion air, of the test engine, and the retention time of the combustion mixture, particularly in the form of a characteristic number calculated therefrom, are taken into consideration.

4. The method according to one or more of the preceding claims, **characterized in that** prior to the comparison the pressure signals are filtered by means of a signal filter having a band pass, particularly in the range of 3 to 15 kHz, and/or having a high pass above 3 kHz.

5. The method according to one or more of the preceding claims, **characterized in that** the ignition time and the start of knocking of the combustion mixture of fuel and combustion gas, particularly of combustion air, are captured, particularly by means of a sensor, and the time difference between said times and/or the difference of the respective test engine crank angles are taken into consideration.

6. The method according to one or more of the preceding claims, **characterized in that** the pressure signals are compared with each other on the basis of knock pressure amplitude, peak pressure, pressure rise rate, frequency and/or knocking intensity.

7. The method according to one or more of the preceding claims, **characterized in that** the combustion curve of a single working cycle or a plurality of working cycles, particularly averaged, is determined and the ignition delay, preignition, maximum combustion rate and/or the residual quantity of combustion mixture of fuel and combustion gas, particularly combustion air, are determined at the time that knocking starts and are taken into consideration in the characterization of the knock resistance.

8. The method according to one or more of the preceding claims, **characterized in that** the combustion curve of a single working cycle or a plurality of working cycles of the test engine is determined and at least one previously defined value of the combustion curve, such as the start of combustion, is determined by means of at least one sensor, particularly a sensor for measuring the ion current within the test engine, a sensor for measuring the structure-bome sound of the test engine, and/or an optical sensor and is taken into consideration when characterizing the knock resistance.

9. The method according to one or more of the preceding claims, **characterized in that** the characterization of the knock resistance comprises a statistical analysis of the pressure signals, wherein the statistical analysis comprises the determination of the frequency distribution of the pressure signals, knock pressure amplitudes, peak pressures, pressure rise rates, frequency, and/or knocking intensity.

10. The method according to one or more of the preceding claims, **characterized in that** the time curve of the crank angle of the test engine during combustion is determined by means of a crank angle sensor.

11. The method according to one or more of the preceding claims, **characterized in that** the compression ratio of the test engine is calculated on the basis of the cylinder pressure at a defined crank angle.

12. The method according to one or more of the preceding claims, **characterized in that** the pressure signals are compared with each other taking into consideration the mass of the injected fuel and/or of the combustion gas, particularly the combustion air, in the cylinder, taking into consideration the intake temperature of the combustion gas and/or of the mixture of combustion gas and fuel, taking into consideration the intake pressure of the combustion gas and/or of the mixture of combustion gas and fuel and/or taking into consideration the revolution rate of the test engine.

13. The method according to one or more of the preceding claims, **characterized in that** the determined pressure signals, particularly as a function of the crank angle and/or the compression ratio, are recorded in a database.

## Revendications

1. Procédé pour la caractérisation du pouvoir antidétonant d'un carburant à l'aide d'un moteur d'essai, dans lequel
- la variation, dans le temps, de la pression de cylindre du moteur d'essai durant la combustion du carburant dans le moteur d'essai est détectée séparément pour plusieurs cycles de travail du moteur d'essai, particulièrement à l'aide d'un capteur de pression piézoélectrique,
- les allures de pression déterminées sont analysées séparément par rapport à un paramètre caractéristique défini, par exemple l'amplitude de pression maximale, **caractérisé en ce que**
- les paramètres caractéristiques respectifs des différents cycles de travail sont analysés par rapport à leur distribution de fréquences,
- sur la base de la répartition de fréquences déterminée, les signaux de pression détectés sont comparés avec les signaux de pression correspondants d'au moins un carburant normalisé d'un pouvoir antidétonant connu et
- partant de la comparaison des signaux de pression, au moins un paramètre caractéristique est déterminé, qui sert de mesure pour le pouvoir antidétonant du carburant.

2. Procédé selon la revendication 1, **caractérisé en ce que** le carburant normalisé utilisé est de l'isooctane et/ou du n-heptane.

3. Procédé selon l'une ou plusieurs des revendications précédentes, **caractérisé en ce que** la pression et/ou la température du mélange de combustion de carburant et de gaz de combustion, particulièrement de l'air de combustion, du moteur d'essai ainsi que la durée de séjour du mélange de combustion, particulièrement sous la forme d'un chiffre caractéristique calculé à partir de ces valeurs, sont pris en compte.

4. Procédé selon l'une ou plusieurs des revendications précédentes, **caractérisé en ce que** les signaux de pression sont filtrés avant la comparaison à l'aide d'un filtrage de signal avec filtre passe-bande, particulièrement dans la plage de 3 à 15 kHz et/ou avec filtre passe-haut à partir de 3 kHz.

5. Procédé selon l'une ou plusieurs des revendications précédentes, **caractérisé en ce que** le point d'allumage ainsi que le début de la détonation du mélange de combustion de carburant et de gaz de combustion, particulièrement l'air de combustion, sont détectés, particulièrement à l'aide d'un capteur, et la différence de temps intermédiaire et/ou la différence entre les différents angles de vilebrequin du moteur d'essai sont prises en compte.

6. Procédé selon l'une ou plusieurs des revendications précédentes, **caractérisé en ce que** les signaux de pression sont comparés les uns avec les autres sur la base de leur amplitude de pression de détonation, de leur pression de crête, de leur vitesse de montée en pression, de leur fréquence et/ou de leur intensité de détonation.

7. Procédé selon l'une ou plusieurs des revendications précédentes, **caractérisé en ce que** l'allure de la combustion d'un seul ou de plusieurs, particulièrement moyennés, cycles de travail du moteur d'essai, est déterminée et, à partir de cette base, le retard à l'allumage, le pré-allumage, la vitesse de combustion maximale et/ou la quantité résiduelle du mélange de combustion de carburant et de gaz de combustion, particulièrement d'air de combustion, sont déterminés au moment du début de la détonation et pris en compte lors de la caractérisation du pouvoir antidétonant.

8. Procédé selon l'une ou plusieurs des revendications précédentes, **caractérisé en ce que** l'allure de la combustion d'un seul ou de plusieurs cycles de travail du moteur d'essai est déterminée et au moins une valeur d'allure de combustion définie au préalable, telle que, par exemple, le début de la combustion, est déterminée à l'aide d'au moins un capteur, particulièrement un capteur pour la mesure du courant ionique au sein du moteur d'essai, un capteur pour la mesure des bruits de structure du moteur d'essai et/ou un capteur optique et prise en compte lors de la caractérisation du pouvoir antidétonant.

9. Procédé selon l'une ou plusieurs des revendications précédentes, **caractérisé en ce que** la caractérisation du pouvoir antidétonant comprend une évaluation statistique des signaux de pression, sachant que l'évaluation statistique comprend la détermination de la répartition de fréquences des signaux de pression, des amplitudes de pression de détonation, des pressions de crête, des vitesses de montée en pression, de la fréquence et/ou de l'intensité de détonation.

10. Procédé selon l'une ou plusieurs des revendications précédentes, **caractérisé en ce que** l'allure dans le temps de l'angle de vilebrequin du moteur d'essai durant la combustion est déterminée à l'aide d'un capteur d'angle de vilebrequin.

11. Procédé selon l'une ou plusieurs des revendications précédentes, **caractérisé en ce que** le taux de compression du moteur d'essai est calculé sur la base de la pression dans le cylindre à un angle de vilebrequin défini.

12. Procédé selon l'une ou plusieurs des revendications précédentes, **caractérisé en ce que** les signaux de pression sont comparés les uns avec les autres compte tenu de la masse du carburant et/ou du gaz de combustion injecté, particulièrement de l'air de combustion injecté dans le cylindre, compte tenu de la température d'aspiration du gaz de combustion et/ou du mélange de gaz de combustion et de carburant, compte tenu de la pression d'aspiration du gaz de combustion et/ou du mélange de gaz de combustion et de carburant et/ou compte tenu du régime du moteur d'essai.

13. Procédé selon l'une ou plusieurs des revendications précédentes, **caractérisé en ce que** les signaux de pression déterminés sont enregistrés dans une base de donnée particulièrement en fonction de l'angle de vilebrequin et/ou du taux de compression.
